# EUROPEAN PATENT SPECIFICATION

(11) **EP 2 052 754 B1**
(45) Date of publication and mention of the grant of the patent: **20.06.2018**
(21) Application number: 08161871.2
(22) Date of filing: 05.08.2008
(51) Int. Cl.: A61M 16/22, A61M 16/08, A62B 19/00, A62B 9/04

(54) **Disposable absorber having an adapter and a lip seal**
Wegwerfabsorber mit einem Adapter und einer Lippendichtung
Absorbeur jetable disposant d'un adaptateur et d'un joint à lèvre

(30) Priority: 24.10.2007 DE 102007050853
(43) Date of publication of application: 29.04.2009
(60) Divisional application: 17205818.2
(73) Proprietor: Drägerwerk AG & Co. KGaA, 23558 Lübeck (DE)
(72) Inventor: Kleinschmidt, Lothar, 23628, Krummesse (DE)
(74) Representative: Haseltine Lake LLP

(56) References cited:
- EP-A1- 1 748 232
- WO-A1-01/51841
- DE-B3-102004 020 133
- DE-B3-102007 025 809
- GB-A- 959 508
- US-A- 2 918 356

## Description

The invention relates to a combination of adapter and absorber container including an adapter for adapting an absorber container to a breathing system, having a first gas passage and a second gas passage which extend into the region of the connection between the absorber container and the adapter, and having seals at the first gas passage and the second gas passage in the region of the connection between the absorber container and the adapter.

In anaesthetic apparatus and also in diving equipment, absorber containers filled with soda lime are used to bind the CO₂ (carbon dioxide) contained in the exhaled air.

Disposable absorbers are often used. The use of a disposable absorber produces the following advantages for the user: direct contact with the soda lime is avoided when the absorber container is being handled and there is no nuisance caused by dust, There is also an improvement in the completeness with which the absorber filling is used and a cost saving is thus achieved, because a change of absorber is possible at any time, such as even during an operation for example, and the absorber can thus be used until the filling is completely exhausted. As an alternative, use is also made of refillable absorber containers. Refilling then preferably takes place through the bottom of an absorber container or its cover or laterally through its circumferential surface.

The absorber container needs to be quickly and easily replaceable without causing any interference with respiration. An example of an absorber of the kind specified in the opening paragraph can be seen from DE 197 29 739 A1.

An absorber container is usually fitted to the existing absorber receptacle of a breathing system by means of an adapter and if required an intermediate plate. The interface at the absorber receptacle of the breathing system or the interface at the intermediate plate forms for example, together with the interface at the adapter, a bayonet connection. Once the two interfaces have been inserted one in the other and have then been turned relative to one another, the breathing system and the adapter are clamped together with a gas-tight seal. The absorber container is preferably connected to the adapter by means of a pivotable receiving plate having an integral snap-action closure. For this purpose, the receiving plate has for example guide grooves to receive corresponding guide ribs on the absorber container. If the receiving plate can be pivoted to a position in which it is inclined at about 30° to the horizontal, then the absorber container automatically slides to its end position, which is defined by a recess milled in the receiving plate. By pivoting the absorber container and the receiving plate towards the adapter, the snap-action closure is caused to close. It is particularly important in this case for there to be a gas-tight connection between the adapter and the absorber container.

Known in this connection are gaskets which are hooked into the adapter and which are held in place on a cylindrical inner bush in the adapter by undercuts. The gaskets make a planar, butt seal at an end face against cylindrical stub tubes on the absorber containers, which are also referred to as sealing "seats". Unevennesses, contamination in the form of lime dust, or damage, at the end-faces of the sealing seats may cause leaks.

There are also internal gaskets on a spring-loaded body acting as a valve which, during a locking operation, makes the connection for gas between the absorber container and the breathing system. By, for example, pressing with a finger on a specially marked point on the receiving plate, the absorber container is unlocked and the connection for gas between the breathing system and the absorber container is broken. At the same time, a bypass is created within the adapter by means of the spring-loaded valve body acting as a valve and the connection for gas to the surroundings is broken. In this position, respiration takes place without the absorber being in the circuit.

A further difficulty lies in the fact that high forces are required to lock the adapter and the absorber container, because tolerances on the dimensions of the individual components have to be taken up by deforming the gaskets to the appropriate degree. This causes increased wear and, as a result, premature failure of the gaskets.

The present invention provides a combination of adapter and absorber container characterized by the features of the characterising portion of claim 1. Optional features are recited in the dependent claims.

Embodiments of he present invention provide an absorber container and an adapter of the kind mentioned in which the connecting system between the breathing system and the absorber container allows a gas-tight connection to be made easily and in such a way that leaks are avoided at the seals of the connecting system.

The adapter for adapting an absorber container to a breathing system may comprise:
- a first and a second gas passage which extend into the region of the connection between the absorber container and the adapter,
- a valve means along the run of the gas passages which has a seal which either seals off the gas passages from the valve means in such a way that the absorber container is cut off from the run of the gas passages, or which seals off the gas passages from the valve means in such a way that the absorber container is in the run between the first and second gas passages, and is characterised in that
- the seal has sealing surfaces which extend at an angle to the end-face of the absorber container.

What is meant by "at an angle to the end-face of the absorber container" in connection with the invention is at an angle of more than 10°, and preferably of more than 30°, and as a particular preference of more than 45°, to the end-face of the absorber container.

The advantage of the adapter of the invention lies in essence in its sealing properties, which are improved because an inner and/or outer valve seat for example on the absorber container not only bears against a seal at the end-face but because sealing surfaces are also formed by a further portion of surface, e.g. a circumferential surface of the valve seat which is perpendicular to the end-face of the absorber container, and/or a depressed surface which is obtained as an extension of the valve seat, take the form of sealing surfaces. The sealing surface thus comprises a plurality of portions of surface which are inclined to, or offset from, one another, which gives improved sealing properties.

The portions of surface may also be divided in such a way that a first portion of surface is the end-face of a valve seat and a further portion of surface is situated on the circumferential surface of the valve seat. It is also within the scope of the invention, and is a particular preference, for there to be provided on the circumferential surface a plurality of portions of surface which have individual sealing lips associated with them.

In a preferred embodiment, the first gas passage takes the form of an inner gas passage and the second gas passage takes the form of an outer gas passage which is arranged to be concentric with the latter.

It has also proved advantageous for the sealing surface to comprise a sealing seat which extends at the end-face relative to the absorber container, and a sealing lip which bears against the circumferential surface of the sealing seat.

The sealing seat on the absorber container is in particular a valve seat belonging to the valve means. In this case, an annular inner valve seat has for example a mean diameter of 22 mm (millimetres) and inside and outside diameters of 20 and 24 mm respectively. An annular outer valve seat has for example a mean diameter of 42 mm and the inside and outside diameters are 40 and 44 mm respectively. Measured from the top face of the absorber container, the height of the seat is preferably 5.5 mm. The depressed surface follows on in an annular form on the outside and is preferably situated 2.5 mm below the top face of the absorber container, which means that the height of the seat from the depressed surface is 8 mm. The depressed surface has a inside diameter of 44 mm and an outside diameter of 60.5 mm, and the outer circumferential surface of the depressed surface is preferably inclined, in the region of the outside diameter, at 60° to the end-face of the absorber container.

The sealing lips may bear against a circumferential surface of the sealing seat and/or against an end-face formed by a depressed surface and/or against the end-face of a sealing seat.

In principle, it is possible for different seals to be combined in the case of an adapter or an absorber container. In this way, a plurality of lip seals for example may bear against an outer sealing seat and an inner sealing seat may be part of a conventional gasket and may form a sealing surface at an end-face.

If necessary, it is possible for seals having sealing lips made of elastomers not to be used if for example a spring-loaded body acting as a valve and a valve seat whose surface is of a suitable nature butt against one another and the conditions of pressure on the two sides of the seal do not put the seal under any great stress. What also shows itself to be particular advantageous is for the sealing seat to be of an annular form having rounded edges and for more than one lip seal to bear against the circumferential surface of a sealing seat. In particular, a depressed surface is formed as an extension, to produce an end-face relative to the absorber container, to allow a plurality of lip seals to be fitted against the latter.

The object of the invention is achieved by a combination of adapter and absorber container for adaption to a breathing system which comprises an adapter as described above.

An embodiment of the invention will be explained in what follows by reference to the accompanying drawings. In the drawings:
Fig. 1 is a diagrammatic schematic view in longitudinal section of an absorber container having a connected adapter, in the locked position,
Fig. 2 is a diagrammatic schematic view in longitudinal section of the absorber container having an adapter of Fig. 1, in the pivoted-away position,
Fig. 3 is a diagrammatic schematic plan view of the absorber container shown in Fig. 1, showing the inner and outer valve seats,
Fig. 4 is a diagrammatic schematic view in cross-section of part of the absorber container shown in Fig. 1, looking in the direction B indicated in Fig. 3,
Figs. 5 to 9 are diagrammatic views in profile of various sealing rings, and
Fig. 10 is a diagrammatic schematic view in cross-section of part of the absorber container, showing a sealing ring in profile.

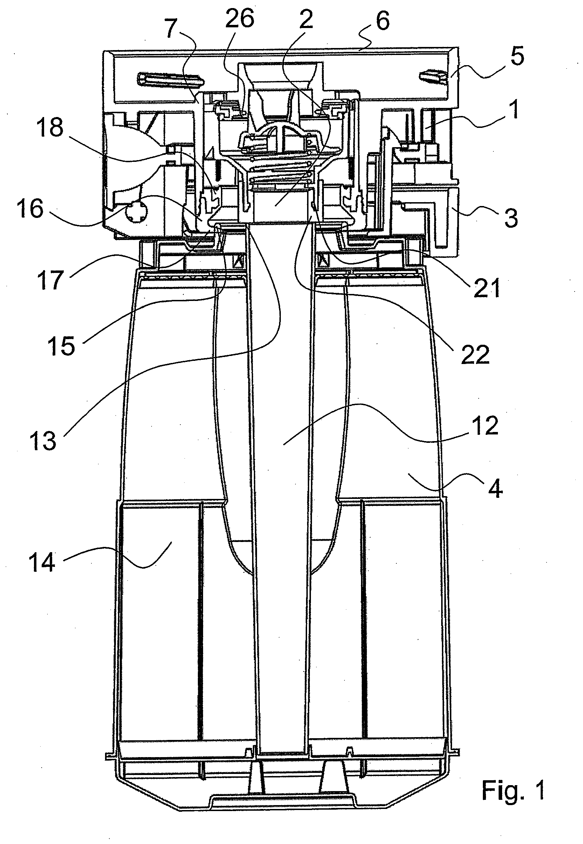

Fig. 1 is a schematic view in longitudinal section of an absorber container 4 having a connected adapter 1, in the locked position. The adapter 1 has a main body 5 having a connecting cup 6 for connection to a breathing system which is not shown in Fig. 1. To connect the absorber container 4 to the adapter 1, the absorber container 4 is inserted in a receptacle 3 and pivoted towards the adapter 1, which has already been done in Fig. 1. The absorber container 4 has an inner gas passage 12 having an inner valve seat 13 and an outer gas passage 14, arranged to be concentric with the inner gas passage 12, having an outer valve seat 15. The inner gas passage 12 defines the path of flow from the breathing system to the absorber container 4 when the connected adapter 1 is in the locked position, and the outer gas passage 14 defines the path of flow from the absorber container 4 back to the breathing system when the connected adapter 1 is in the locked position.

Within the adapter 1, the inner gas passage 12 extends through the interior of a valve means 2. Situated at the underside of a guiding sleeve 7 to receive the valve means 2, there is a first sealing ring 16 which has an outer sealing lip 17 directed towards the absorber container 4 and an inner sealing lip 18. A portion 21 of the wall of the housing of the valve means 2 is provided with a second sealing ring 22 at its free end which extends towards the absorber container 4.

When the absorber container 4 is connected to the adapter 1, the outer sealing lip 17 of the first sealing ring 16 rests on the outer valve seat 15, and the second sealing ring 22 rests on the inner valve seat 13.

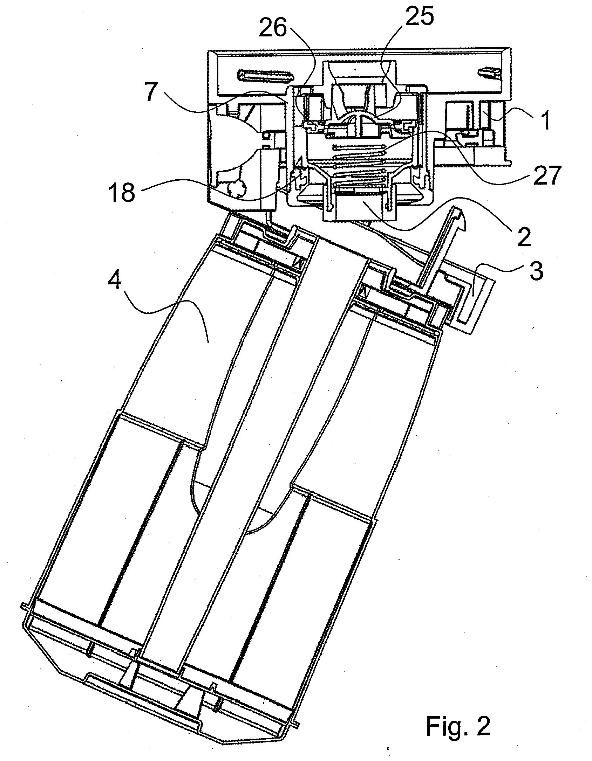

Fig. 2 is a schematic view in longitudinal section of the absorber container 4 and adapter 1 of Fig. 1 in the pivoted-away position. The same reference numerals will be used in what follows for the same components as in Fig. 1.

Within the adapter 1, the path of flow extends through the interior of the valve means 2. The inner sealing lip 18 of the first sealing ring 16 bears from the outside against the housing of the valve means 2. The inner sealing lip 18 and the housing of the valve means 2 form a sealing region to stop the flow of gas from the absorber container 4 when the absorber container 4 is not connected to the adapter 1. A sealing lip 26 bears against a body 25 acting as a valve at the top end of the valve means 2. The sealing lip 26 and the body 25 acting as a valve, which latter pressed against the sealing lip 26 by a compression spring 27, form a further sealing region to stop the flow of gas to the absorber container 4. When the absorber container 4 is in the position shown in Fig. 2, the path for flow extends via the inner gas passage 12 and through openings within the adapter 1 to the outer passage 14, without following the path through the absorber container 4.

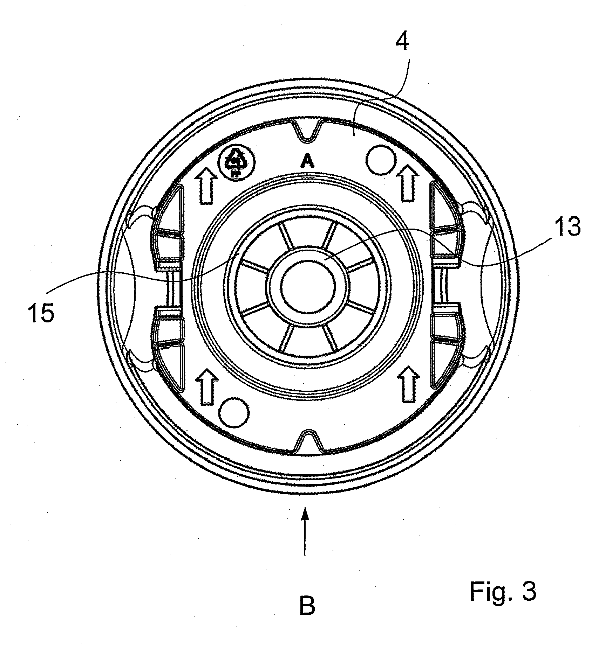

Fig. 3 is a schematic plan view of the absorber container 4 showing the inner valve seat 13 and the outer valve seat 15.

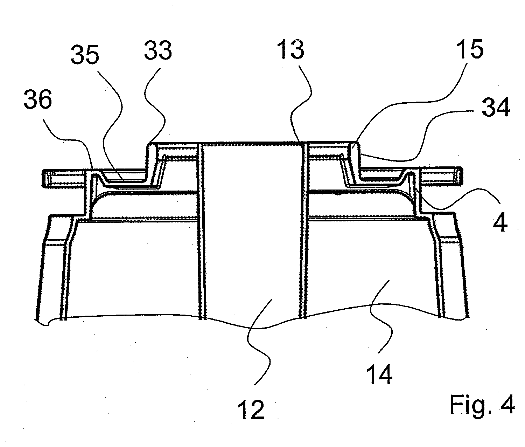

Fig. 4 is a schematic view in cross-section of part of the absorber container 4, looking in the direction indicated by B in Fig. 3, showing the inner valve seat 13 and the outer valve seat 15. The outer valve seat 15 has an end-face 33 and a circumferential surface 34, from which latter a depressed surface 35 follows on in an annular form in the outward direction, on the top face 36 of the absorber container 4. In the region of the outer circumference, the outer circumferential surface of the depressed surface 35 is inclined at 60° to the top face 36 of the absorber container.

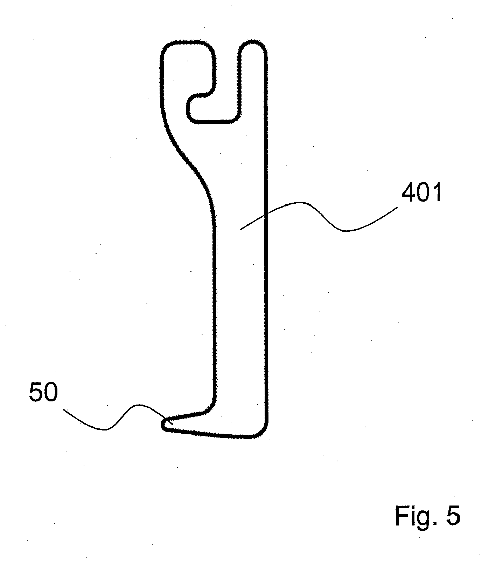

Figs. 5 to 9 are views in profile of various sealing rings 401, 402, 403, 404, 405 having sealing lips 50, 51, 52, 53, 54. The sealing rings 401, 402, 403, 404, 405 are first sealing rings 16. The sealing lips 50, 51, 52, 53, 54 are outer sealing lips 17.

Fig. 5 shows in profile a sealing ring 401 having a sealing lip 50 situated at the bottom.

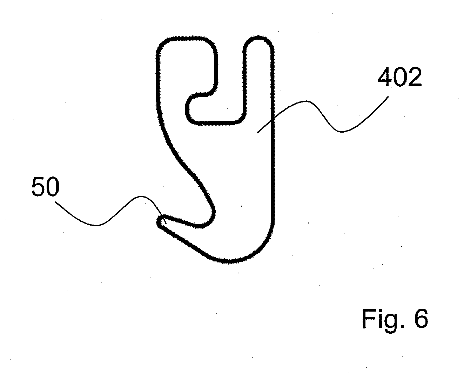

As an alternative, Fig. 6 shows in profile a sealing ring 402 having a sealing lip 50 situated towards the top.

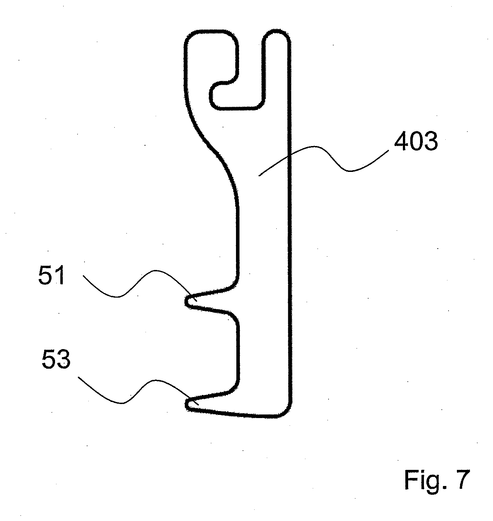

Fig. 7 shows in profile a further variant, which is a sealing ring 403 having an annular sealing lip 51 situated towards the top and an annular sealing lip 53 situated at the bottom.

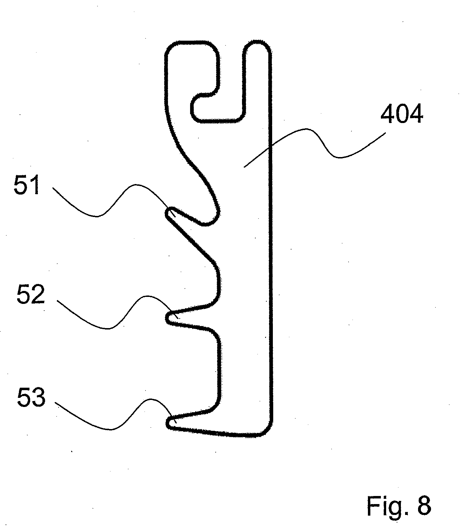

Fig. 8 shows in profile an additional variant, which is a sealing ring 404 having a sealing lip 51 situated towards the top, a sealing lip 52 situated in the centre and a sealing lip 53 situated at the bottom. The sealing lips 51, 52, 53 all bear against the circumferential surface 34 of the outer valve seat 15.

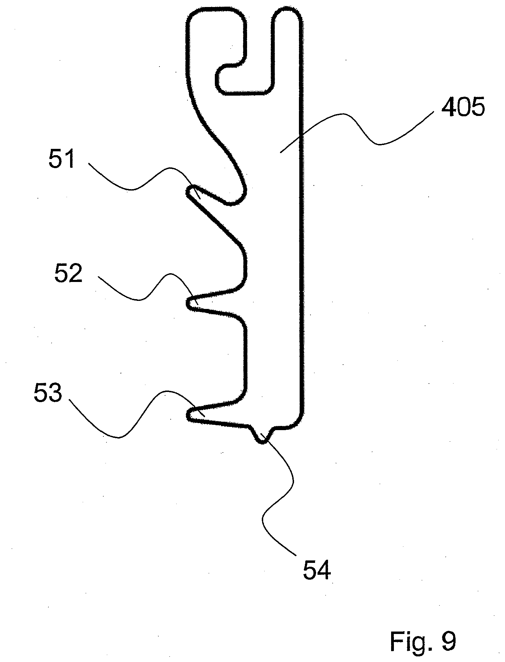

Fig. 9 shows in section a further variant, which is a sealing ring 405 having a sealing lip 51 situated towards the top, a sealing lip 52 situated in the centre, a sealing lip 53 situated at the bottom and a sealing lip 54 situated underneath, which sealing lips are each annular in form.

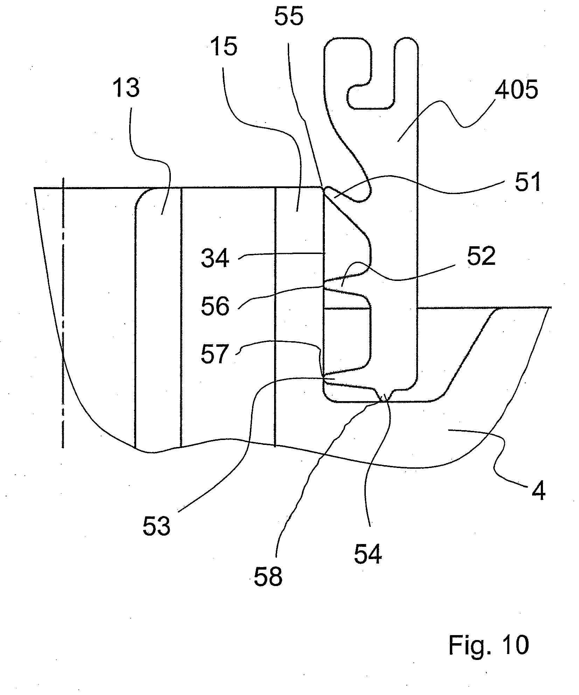

Fig. 10 shows the sealing ring 405 of Fig. 9 when co-operating with the outer valve seat 15 of the absorber container 4. The sealing lips 51, 52, 53 bear in this case against portions 55, 56, 57 of the circumferential surface 34 of the outer valve seat 15. The underneath sealing lip 54 is situated against a further portion of surface 58 within the depressed surface 35.

## Claims

1. A combination of an adapter (1) and an absorber holder (4), wherein the adapter (1) is for adapting the absorber holder (4) to a breathing system:
the absorber holder (4) having a first gas passage (12) and a second gas passage (14) which extend into the region of the connection between the absorber holder (4) and the adapter (1), wherein the second gas passage (14) comprises an outer valve seat (15) having an outer circumferential surface (34) intended to serve as a sealing surface;
the adapter (1) having seals at the first gas passage (12) and the second gas passage (14) in the region of the connection between the absorber holder (4) and the adapter (1), wherein the seal (16, 401, 402, 403, 404, 405) for the second gas passage (14) comprises sealing surfaces (34, 55, 56, 57) in the form of at least one annular sealing lip which extends around the circumferential surface (34) of the outer valve seat (15), and which extends into the circumferential region and seals against the circumferential surface (34) of the second gas passage (14).

2. A combination of an adapter (1) and an absorber holder (4) according to claim 1, **characterised in that** the first gas passage (12) takes the form of an inner gas passage and the second gas passage (14) takes the form of an outer gas passage which is arranged to be concentric with the latter.

3. A combination of an adapter (1) and an absorber holder (4) according to claim 1 or 2, **characterised in that** a plurality of sealing lips (17, 51, 52, 53) which are arranged at a distance from one another and which bear against portions (55, 56, 57) of the circumferential surface (34) are provided in the region of the circumferential surface (34).

4. A combination of an adapter (1) and an absorber holder (4) according to one of claims 1 to 3, **characterised in that** a depressed surface (35) below the valve seat (15) of the outer gas passage (14) is present as a further sealing surface.

5. A combination of an adapter (1) and an absorber holder (4) according to claim 4, **characterised in that** the seal (405) has a further sealing lip (54) in the region of the depressed surface (35).

## Patentansprüche

1. Kombination aus Adapter (1) und Absorberhalter (4), wobei der Adapter (1) dazu dient, den Absorberhalter (4) an ein Patientensystem anzupassen:
wobei der Absorberhalter (4) einen bis in den Verbindungsbereich von Absorberhalter (4) und Adapter (1) reichenden ersten Gasdurchgang (12) und zweiten Gasdurchgang (14) aufweist, wobei der zweite Gasdurchgang (14) einen äußeren Ventilsitz (15) mit einer als Dichtfläche dienenden äußeren Umfangsfläche (34) umfasst;
wobei der Adapter (1) im ersten Gasdurchgang (12) und im zweiten Gasdurchgang (14) im Verbindungsbereich von Absorberhalter (4) und Adapter (1) Dichtungen aufweist, wobei die Dichtung (16, 401 402, 403, 404, 405) für den zweiten Gasdurchgang (14) Dichtflächen (34, 55, 56, 57) in Form von mindestens einer ringförmigen Dichtlippe aufweist, die sich um die Umfangsfläche (34) des äußeren Ventilsitzes (15) und in den Umfangsbereich erstreckt und gegen die Umfangsfläche (34) des zweiten Gasdurchgangs (14) abdichtet.

2. Kombination aus Adapter (1) und Absorberhalter (4) gemäß Anspruch 1, **dadurch gekennzeichnet, dass** der erste Gasdurchgang (12) die Form eines inneren Gasdurchgangs und der zweite Gasdurchgang (14) die Form eines äußeren Gasdurchgangs aufweist und zu letzterem konzentrisch angeordnet ist.

3. Kombination aus Adapter (1) und Absorberhalter (4) gemäß Anspruch 1 oder 2, **dadurch gekennzeichnet, dass** eine Vielzahl von Dichtlippen (17, 51, 52, 53) beabstandet angeordnet sind und an im Bereich der Umfangsfläche (34) vorgesehenen Abschnitten (55, 56, 57) anliegen.

4. Kombination aus Adapter (1) und Absorberhalter (4) gemäß einem der Ansprüche 1 bis 3, **dadurch gekennzeichnet, dass** unterhalb des Ventilsitzes (15) des äußeren Gasdurchgangs (14) eine vertiefte Fläche (35) als weitere Dichtfläche vorgesehen ist.

5. Kombination aus Adapter (1) und Absorberhalter (4) gemäß Anspruch 4, **dadurch gekennzeichnet, dass** die Dichtung (405) im Bereich der vertieften Fläche (35) eine weitere Dichtlippe (54) aufweist.

## Revendications

1. Une combinaison d'un adaptateur (1) et d'un support d'absorbeur (4), dans laquelle l'adaptateur (1) sert à adapter le support d'absorbeur (4) à un système respiratoire :
le support d'absorbeur (4) ayant un premier passage de gaz (12) et un second passage de gaz (14) qui s'étendent dans la région de connexion entre le support d'absorbeur (4) et l'adaptateur (1), dans laquelle le second passage de gaz (14) comprend un siège de soupape externe (15) ayant une surface circonférentielle externe (34) destinée à servir de surface d'étanchéité ;
l'adaptateur (1) comportant des joints au premier passage de gaz (12) et au second passage de gaz (14) dans la région de connexion entre le support d'absorbeur (4) et l'adaptateur (1), dans laquelle le joint (16, 401, 402, 403, 404, 405) pour le second passage de gaz (14) comprend des surfaces d'étanchéité (34, 55, 56, 57) sous la forme d'au moins une lèvre d'étanchéité annulaire qui s'étend autour de la surface circonférentielle (34) du siège de soupape externe (15), et qui s'étend dans la région circonférentielle et assure l'étanchéité contre la surface circonférentielle (34) du second passage de gaz (14).

2. Une combinaison d'un adaptateur (1) et d'un support d'absorbeur (4), selon la revendication 1, **caractérisée en ce que** le premier passage de gaz (12) prend la forme d'un passage de gaz interne et le second passage de gaz (14) prend la forme d'un passage de gaz externe qui est agencé pour être concentrique avec ce dernier.

3. Une combinaison d'un adaptateur (1) et d'un support d'absorbeur (4), selon la revendication 1 ou 2, **caractérisée en ce que** plusieurs lèvres d'étanchéité (17, 51, 52, 53) qui sont disposées à distance l'une de l'autre et qui sont en appui contre des parties (55, 56, 57) de la surface circonférentielle (34) sont fournies dans la région de la surface circonférentielle (34).

4. Une combinaison d'un adaptateur (1) et d'un support d'absorbeur (4) selon l'une des revendications 1 à 3, **caractérisée en ce qu'**une surface abaissée (35) sous le siège de soupape (15) du passage de gaz extérieur (14) est présente comme une autre surface d'étanchéité.

5. Une combinaison d'un adaptateur (1) et d'un support d'absorbeur (4), selon la revendication 4, **caractérisée en ce que** le joint (405) comporte une autre lèvre d'étanchéité (54) dans la région de la surface abaissée (35).
